# EUROPEAN PATENT APPLICATION

(11) **EP 1 426 403 A1**
(43) Date of publication of application: **09.06.2004**
(21) Application number: 02751675.6
(22) Date of filing: 25.07.2002
(51) Int. Cl.: C08J 5/18, A61F 13/514, C08L 29/04

(54) **WATER−DISINTEGRABLE RESIN FILM, LAMINATED WATER−DISINTEGRABLE RESIN FILM, WATER−DISINTEGRABLE COMPOSITE LAMINATE AND METHOD FOR PRODUCTION THEREOF**

(30) Priority: 30.07.2001 JP 2001230121; 12.12.2001 JP 2001378942
(71) Applicant: SEKISUI CHEMICAL CO., LTD., Osaka-shi, Osaka 530-8565 (JP)
(72) Inventor: NAKAGAWA, Hiroaki, SEKISUI CHEMICAL CO., LTD., Kyoto-shi, Kyoto 601-8105 (JP); SATO, Kazuhiro, SEKISUI FILM CO., LTD., Chita-gun, Aichi 470-2196 (JP); SHIGEMITSU, Takahiro, SEKISUI FILM CO., LTD., Chita-gun, Aichi 470-2196 (JP); FUKAYA, Mitsuharu, SEKISUI FILM CO., LTD., Chita-gun, Aichi 470-2196 (JP); KANDO, Shin'ichi, SEKISUI FILM CO., LTD., Chita-gun, Aichi 470-2196 (JP); HASHIMOTO, Yosei, SEKISUI FILM CO., LTD., Watari-gun, Miyagi 989-2324 (JP)
(74) Representative: Keltie, David Arthur
(86) International application number: PCT/JP2002/007533
(87) International publication number: WO 2003/011953

(57) **Abstract**

A water-disintegrable resin film, characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united. The water-disintegrable resin film has a predetermined mechanical strength under a wet and warm or hot condition and also exhibits excellent disintegradability for a large amount of water, and thus can be suitably used as a back sheet of a liner for sanitary goods, which requires the combination of good water resistance and good water-disintegradability.

## Description

### TECHNICAL FIELD

The present invention relates to a water-disintegrable resin film, a laminated water-disintegrable resin film and a water-disintegrable composite laminate which are each suitably used as a back sheet of a liner for sanitary goods such as sanitary napkin liner, liner for vaginal discharge and liner for incontinence, and a method for production thereof.

### BACKGROUND ART

Conventionally, a liner for sanitary goods has been widely utilized. The liner for sanitary goods is generally produced by overlapping a liquid-permeable top sheet and a liquid-impermeable back sheet with each other, integrating opposing outer peripheries of the sheets, putting a liquid absorbent in a space between opposing faces of the top sheet and the back sheet and, further, forming an adhesive layer for detachably fixing the liner for sanitary goods to an underwear on the outer face of the back sheet.

The liner for sanitary goods is disposed after use. In this connection, since many non-water-disintegrable materials are used in the conventional liner for sanitary goods and non-water-disintegrable materials are often used particularly as a back sheet that requires a leakage prevention property, the liner for sanitary goods has a problem in that, when flushed in a toilet, the non-water-disintegrable materials cause the choking of the pipes.

Specifically, as a liner for sanitary goods, Japanese Unexamined Patent Publication No. 2000-336197 proposes a liner for sanitary goods which uses a polyolefin-based resin film as the back sheet. However, since the polyolefin-based resin film is non-water-disintegrable, the liner for sanitary goods is associated with the problem that, when flushed after use in a toilet, the liner for sanitary goods causes the choking of the pipes or places a heavy load on a septic tank even if the liner for sanitary goods reaches the septic tank.

For this reason, as a back sheet material of the liner for sanitary goods, free from the choking of the pipes even if flushed after use in a toilet, for example, Japanese Published Patent Publication No. 5-509045 or Japanese Published Patent Publication No. 10-511902 proposes a laminated film, comprising a water-soluble resin layer whose one face is integrally laminated on a resin layer difficultly soluble in water, to obtain both water-disintegradability and water resistance. Japanese Published Patent Publication No. 7-505059 proposes a disposable article which exhibits an excellent disintegradability for a large amount of water but exhibits water resistance for a small amount of water.

Meanwhile, a liner for sanitary goods is worn normally for about 4 to 5 hours consecutively and the liner for sanitary goods, which is worn, is in a wet and warm or hot condition due to the body temperature and the sweat from the body. In particular, it is not rare that the temperature of the environment in which the liner for sanitary goods is worn reaches 40°C or thereabout in summer.

While being worn by the body, a deforming stress in concert with the motion of the body is always applied to the liner for sanitary goods. Therefore, the liner for sanitary goods need to be free from any fracture such as breakage by the deforming stress applied under the wet and warm or hot condition.

However, the above-mentioned laminated film and disposable article are not designed by taking into consideration the use in the wet and warm or hot condition under constant deforming stress applied. Further, the end face at the outer periphery of the back sheet in the liner for sanitary goods is exposed to exterior, that is, exposed directly to the wet and warm or hot condition. As a result, the problem is that, when the above-mentioned laminated film and disposable article are used as the back sheet of the liner for sanitary goods under a wet and warm or hot condition, the mechanical strength such as tensile strength is remarkably reduced to an extent that fracture such as break due to deforming stress occurs.

As described above, a liner for sanitary goods is detachably fixed to the underwear by the adhesive layer such that the liner for sanitary goods is removed after use from the underwear. As described above, in the above-mentioned laminated film and disposable article, which are used as a back sheet of the liner for sanitary goods, the mechanical strength of the back sheet is remarkably reduced in a wet and warm or hot environment in which the back sheet is worn.

Therefore, when the liner for sanitary goods is removed from underwear, the back sheet (i.e., the above-mentioned laminated film or disposable article) cannot withstand the adhesion of the adhesive that integrates the back sheet and the underwear and thus the back sheet breaks in the direction of the thickness to thereby cause so-called delamination. This delamination leads to the problem that the back sheet partially remains on the underwear and the underwear is soiled by the adhesive remaining on the underwear.

The present invention provides a water-disintegrable resin film, a laminated water-disintegrable resin film and a water-disintegrable composite laminate which are all excellent in water-disintegradability while having a certain level of water resistance and maintain excellent mechanical strength even when exposed to a wet and warm or hot condition of a high temperature of about 40°C, and provides a method for production thereof.

### DISCLOSURE OF THE INVENTION

A water-disintegrable resin film described in claim 1 is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

That is, if the tensile strength of the water-disintegrable resin film is small, in the case where the water-disintegrable resin film is used as the back sheet of a liner for sanitary goods in a wet and warm or hot condition, the mechanical strength of the water-disintegrable resin film is reduced. Further, if a deforming stress is applied, fracture such as break occurs or delamination occurs, as the back sheet breaks in the direction of thickness, when the liner for sanitary goods is removed after use thereof from underwear. Accordingly, the tensile strength is specified as 2 N/25 mm width or more. On the other hand, the upper limit of the tensile strength is not particularly specified by a value. The larger the tensile strength of the film, the more desirable the film is. However, the film in which this value is large means a film having a large rigidity and therefore does not satisfy the suitability to wear. Accordingly, the tensile strength is preferably 2 to 30 N/25 mm width and a more preferred range is 3 to 20 N/25 mm width.

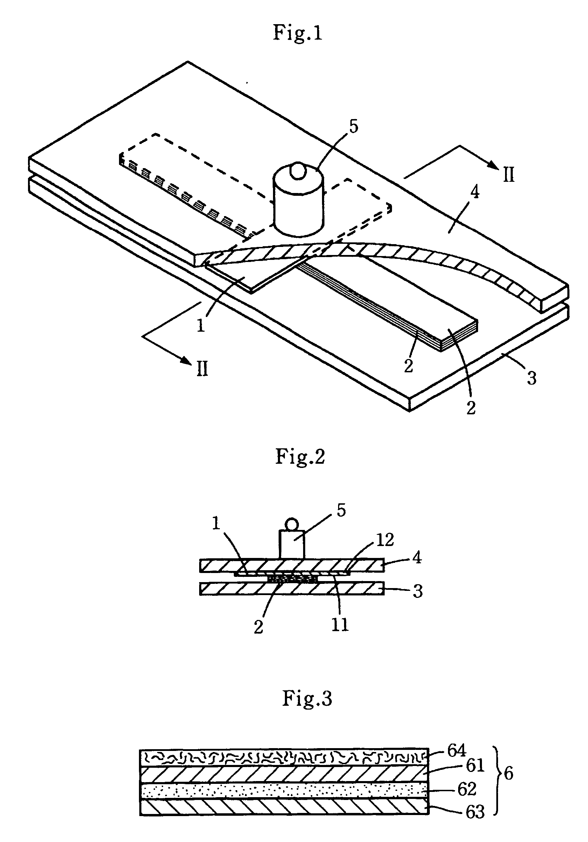

The tensile strength is a value measured by the following procedure. Besides, Fig. 1 is a partially notched perspective view showing a measuring state of the tensile strength. Fig. 2 is a cross-sectional view taken along line II-II of Fig. 1.

First, 10 specimens 1 each having a length of 80 mm × a width of 25 mm are prepared from an arbitrary part of a water-disintegrable resin film. On the other hand, four sheets of cotton gauze 2 each having a length of 180 mm × a width of 20 mm and a basis weight of 37 g/m² are prepared and these four sheets of cotton gauze 2 are completely immersed in water at 40°C. After immersion, these four sheets of cotton gauze 2 are taken out entirely without squeeze (the water content of each sheet of cotton gauze 2 is approximately 2.1 g). These four sheets of cotton gauze 2 are overlapped with one another in a vertical direction and are then gently placed on a horizontal plate 3. Next, the specimen 1 is placed on the cotton gauze 2 such that the directions of lengths of the specimen 1 and the cotton gauze 2 intersect each other at a right angle and the central parts in the direction of length of the specimen 1 and the cotton gauze 2 overlap with each other.

After that, a plate 4 is placed on the specimen 1 and a weight 5 is placed on the plate 4 so that a pressure of 1.47 N/cm² is applied onto the part where the cotton gauze 2 and the specimen 1 overlap with each other.

While the above-described state is maintained, the specimen is allowed to stand for 5 hours under a condition wherein the temperature is 40°C and the relative humidity is 90%, and thereafter the specimen 1 is taken out. Next, under a condition wherein the temperature is 40°C and the relative humidity is 90%, both ends of the specimen 1 are held by a clamp in which the interval between chucks is 60 mm and the specimen 1 is pulled in the length direction at a tensile rate of 200 mm/minute until the specimen 1 breaks. Thus, the tensile strength is measured. Besides, it should be noted that the measurement of the tensile strength is carried out within 5 minutes after the specimen 1 is taken out.

In the measurement, 10 sheets of the specimen 1 were divided into group A and group B which each consisted of 5 sheets of the specimen 1. In the group A, the specimen face 11, which corresponded to one side of the water-disintegrable resin film, faced the cotton gauze 2 side, whereas in the group B, the specimen face 12, which corresponded to the other side of the water-disintegrable resin film, faced the cotton gauze 2 side. After the measurement of the tensile strength, averages were calculated in the group A and in the group B separately. Of these two averages, a larger one was defined as the tensile strength.

The measuring condition described above is based on the assumption that the water-disintegrable resin film is used as the back sheet of a liner for sanitary goods. That is, the condition is that the cotton gauze, after being completely immersed in water at 40°C, was taken out entirely without squeeze and was brought into contact with the specimen 1 for 5 hours under the condition wherein the temperature is 40°C and the relative humidity is 90%. This condition is based on the assumption that in summer, the environment in which the liner for sanitary goods is worn has a temperature of about 40°C and is in a highly wet and warm or hot condition due to the sweat from the body and on the assumption that the average time period for wearing the liner for sanitary goods is about 5 hours. In addition, the pressure of 1.47 N/cm² is based on the presumed weight to be applied to the liner for sanitary goods when a user has a seat.

If the start time for the disintegration of the water-disintegrable resin film by water is long and the water-disintegrable resin film is used as the back sheet of a liner for sanitary goods and the liner for sanitary goods is disposed in a toilet, the liner for sanitary goods tends to choke the pipes of sewage or remains without being water-disintegrated in the sewage facility such as a sewage pipe and a septic tank for a long time thereby placing a large load on the sewage facility. For this reason, the start time for the disintegration of the water-disintegrable resin film by water is specified as 600 seconds or less. On the other hand, if this time is short, even when the water-disintegrable resin film is brought into contact with a small amount of water, the water-disintegrable resin film tends to break. When the water-disintegrable resin film is used as the back sheet of the liner for sanitary goods, the mechanical strength of the water-disintegrable resin film is reduced particularly at a high temperature in summer when the liner for sanitary goods is wet due to perspiration from the body. In such a condition, if an external force is applied, for example, in a sitting condition, the water-disintegrable resin film tends to break. For this reason, the start time for the disintegration of the water-disintegrable resin film by water is preferably 20 to 600 seconds, more preferably 30 to 300 seconds, and most preferably 40 to 180 seconds.

The start time for the disintegration of the water-disintegrable resin film by water is the time needed until the water-disintegrable resin starts to lose its form in a test nearly according to JIS P 4501 for the capability of being united and is more specifically the time measured according to the following method.

First, a beaker having an inner diameter of 7.3 cm and containing 300 cm³ of water at 20±5°C is placed on a magnetic stirrer. The revolution of an discoid rotor having a diameter of 35 mm × a thickness of 12 mm placed in the beaker is adjusted to 600±10 revolutions/minute.

Next, a specimen in the shape of a planar square having a side of 114 mm and taken from an arbitrary part of the water-disintegrable resin film is placed in the beaker and the state of the specimen is visually inspected. Then, the time to be taken for the water-disintegrable resin film to start to lose its form is measured immediately after the specimen is placed in the beaker. According to this procedure, the start time for the disintegration of the water-disintegrable resin film by water is measured with 5 specimens and the average thereof is defined as the start time for the disintegration of the water-disintegrable resin film by water. The term "for the specimen to start to lose its form" means the state where at least part of the specimen starts breaking.

Although the capability of being united is specified by the number of revolution of the discoid rotor in the test according to JIS P 4501 for the capability of being united, the measurement is carried out by the above-mentioned visual inspection. This is because a film-like specimen tends to be curled into a ball or to become entangled with the rotor by the stream of water and thus produces a large deviation if the measurement is carried out by the number of revolution of the discoid rotor.

If the tensile strength at the time of elongating the water-disintegrable resin film by 2% (hereinafter, referred to as "tensile strength at 2% elongation") is small, the water-disintegrable resin film loses its rigidity to an extent that the ease in handling is impaired. On the other hand, if this strength is large, the water-disintegrable resin film imparts a hard feel to an extent that the feeling of wear is impaired when the water-disintegrable resin film is used as the back sheet of a liner for sanitary goods, or the back sheet does not easily deform in compliance with the motion of the human body to an extent that an uncomfortable feeling tends to be created when the liner for sanitary goods is worn. Accordingly, the tensile strength at 2% elongation is preferably 1 to 40 N/25 mm width, more preferably 2 to 30 N/25 mm width, and most preferably 3 to 20 N/25 mm width.

The tensile strength at 2% elongation of the water-disintegrable resin film is measured according to the following method. A specimen having a length of 80 mm × a width of 25 mm is taken from an arbitrary part of the film that has been allowed to stand for 24 hours in the condition wherein the temperature is 23°C and the relative humidity is 50%. On this specimen, a pair of reference lines are written such that the interval between the lines is 20 mm in the length direction and the central part between the lines almost coincides with the central part of the specimen.

Next, while the above-described specimen is maintained in the condition wherein the temperature is 23°C and the relative humidity is 50%, the specimen is held by a clamp in which the distance between chucks is 60 mm and the specimen is pulled in the length direction at a tensile rate of 200 mm/minute. When the interval between the reference lines is elongated by 2%, the tensile strength is measured. According to the procedure described above, the measurement is carried out with 5 specimens and the average is defined as tensile strength at 2% elongation.

The elongation of 2% in the measuring method described above is based on the assumption that the water-disintegrable resin film is used as the back sheet of a liner for sanitary goods. The elongation of 2% is selected from the viewpoint of whether or not the water-disintegrable resin film can be stretched without being broken in compliance with the elongation stress to be generated in accordance with underwear expansion and contraction to be caused by the motion of the person wearing the underwear while the liner for sanitary goods is fixed to the underwear.

The synthetic resin constituting the water-disintegrable resin film is not particularly limited in so far as the film has the tensile strength and the start time for the disintegration of the water-disintegrable resin film by water falling within the above-mentioned ranges. Examples of the synthetic resin include a polyvinyl alcohol-based resin, poly(ethylene-CO-acrylic acid), polyethylene oxide, polypropylene oxide, polyvinylpyrrolidone, polyvinylpyridine, alginate, hydroxypropyl cellulose, gelled starch, a resin based on acrylic acid, and modified products thereof. Among these synthetic resins, a polyvinyl alcohol-based resin is preferable. These synthetic resins may be used singly or in a combination of two or more.

The degree of saponification of the polyvinyl alcohol-based resin is preferably 95 mol% or more, more preferably 97 mol% or more because, if the degree of saponification is low, the water resistance of the water-disintegrable resin film to be obtained becomes lower. If the water-disintegrable resin film is used as the back sheet of a liner for sanitary goods and the environment in which the back sheet is worn becomes a wet and warm or hot condition, the mechanical strength of the water-disintegrable resin film drops to an extent that breakage occurs. The degree of saponification of the polyvinyl alcohol-based resin is one measured nearly according to JIS K 6726.

If the degree of polymerization of the polyvinyl alcohol-based resin is small, the water resistance of the water-disintegrable resin film to be obtained becomes lower. If the water-disintegrable resin film is used as the back sheet of a liner for sanitary goods and the environment in which the back sheet is worn becomes a wet and warm or hot condition, the mechanical strength of the water-disintegrable resin film drops to an extent that breakage occurs, or the mechanical strength of the water-disintegrable resin film drops also in a dry condition. On the other hand, if the degree of polymerization is large, the water-disintegradability of the water-disintegrable resin film to be obtained becomes lower to an extent that, if the water-disintegrable resin film is used as the back sheet of a liner for sanitary goods and the back sheet is disposed in a toilet, the liner for sanitary goods causes the choking of the pipes or remains for a long time without being water-disintegrated in a sewage facility such as a sewage pipe and a septic tank to thereby place a heavy load on the sewage facility. For this reason, the degree of polymerization is preferably 300 to 2000, more preferably 400 to 1300.

Accordingly, when a polyvinyl alcohol-based resin is used as the synthetic resin constituting the water-disintegrable resin film, it is preferable that polyvinyl alcohol-based resin has a degree of saponification of 95 mol% or more and a degree of polymerization of 300 to 2000.

More specifically, examples of the polyvinyl alcohol-based resin include a polymer of a vinyl ester-based monomer, a saponified product thereof, and an oxyalkylene-modified product thereof; and a copolymer of vinyl ester-based monomer and a monomer copolymerizable with this vinyl ester-based monomer, a saponified product thereof (e.g., an ethylene/vinyl alcohol copolymer), and an oxyalkylene-modified product thereof. These may be used singly or in a combination of two or more.

Examples of the vinyl ester-based monomer include vinyl acetate, vinyl propionate, vinyl pivalate, vinyl versate and the like. Among these monomers, vinyl acetate is preferable. Examples of the monomer copolymerizable with the vinyl ester-based monomer include α -olefinic monomers such as ethylene, propylene, 1-butene, 1-pentene and the like, (meth)acrylic acid, (meth)acrylate, (meth)acrylamide, vinyl ether, (meth)acrylonitrile, and the like. In particular, ethylene is preferable.

Further, as to the polyvinyl alcohol-based resin, the use of a combination of a oxyalkylene-modified polyvinyl alcohol-based resin and an ethylene/vinyl alcohol copolymer is preferable from the viewpoint of the ease in adjusting the tensile strength and the start time for the disintegration by water of the obtained water-disintegrable resin film to values in the ranges described above.

Examples of the oxyalkylene-modified polyvinyl alcohol-based resin include ① a resin obtained by the saponification of a copolymer of an unsaturated monomer having an oxyalkylene group and vinyl acetate; ② a resin obtained by a process comprising polymerizing vinyl acetate in the presence of a polyoxyalkylene glycol and saponifying the polymer obtained; ③ a resin obtained by carrying out an addition reaction of an alkylene oxide to polyvinyl alcohol; etc. These resins may be used singly or in a combination of two or more.

The oxyalkylene group is represented by the following formula (1) and a preferred oxyalkylene group is a polyoxyethylene group, a polyoxypropylene group, or a polyoxybutylene group.

-(CR¹H-CR²H-O)ₙ- formula (1)

(in which R¹ and R² each represents hydrogen or an alkyl group and n represents an integer of 1 to 100)

Among the methods of producing the oxyalkylene-modified polyvinyl alcohol-based resin, the method according to ① can be said to be the most preferred method from the viewpoint of ease in designing the molecule. Specific examples of the production of the oxyalkylene-modified polyvinyl alcohol-based resin according to this method include a method in which a (meth)acrylate-type monomer represented by the general formula (2) is copolymerized, a method in which a (meth)acrylamide-type monomer represented by the general formula (3) is copolymerized, a method in which an allyl ether-type monomer represented by the general formula (4) is copolymerized, and a method in which a vinyl ether-type monomer represented by general formula (5) is copolymerized, using these monomers as unsaturated monomers containing an oxyalkylene group.

CH₂=CR-COO-(A-O)ₘ-(CR¹H-CR²H-O)ₙ-H formula (2)

(in which R represents hydrogen or a methyl group; R¹ and R² each represents hydrogen or an alkyl group; A represents an alkylene group, a substituted alkylene group, a phenylene group or a substituted phenylene group; m represents 0 or an integer of 1 or greater; and n represents an integer of 1 to 100)

CH₂=CR-CONR³-(A-O)ₘ-(CR¹H-CR²H-O)ₙ-H formula (3)

(in which R³ represents hydrogen, an alkyl group or
-(A-O)ₘ-(CR¹H-CR²H-O)ₙ-H; and A, R, R¹, R², m and n are the same as those defined above, respectively)

CH₂=CR-CH₂-O-(CR¹H-CR²H-O)ₙ-H formula (4)

(in which R, R¹, R² and n are the same as those defined above, respectively)

CH₂=CH-O-(A-O)ₘ-(CR¹H-CR²H-O)ₙ-H formula (5)

(in which R, R¹, R², m and n are the same as those defined above, respectively)

Among these resins, the oxyalkylene-modified polyvinyl alcohol-based resin, which is obtained by copolymerizing the allyl ether-type monomer represented by the formula (4), is most suitably used.

If the oxyalkylene group content in the oxyalkylene-modified polyvinyl alcohol-based resin is small, the water-disintegradability of the water-disintegrable resin film to be obtained becomes lower to an extent that, when the water-disintegrable resin film is used as the back sheet of a liner for sanitary goods and the back sheet is disposed in a toilet, the liner for sanitary goods tends to choke the sewage pipes or may remain for a long time without being water-disintegrated in a sewage facility such as a sewage pipe and a septic tank to thereby place a heavy load on the sewage facility. On the other hand, if the content is large, the water resistance of the water-disintegrable resin film to be obtained is reduced and the mechanical strength of the water-disintegrable resin film is reduced when the environment in which the back sheet is worn becomes a wet and warm or hot condition due to the heat, sweat and the like from the body during the use of the water-disintegrable resin film as the back sheet of the liner for sanitary goods. In such a condition, if an external force is applied, for example, in a sitting condition, the water-disintegrable resin film tends to break, or, when the liner for sanitary goods is removed after use from the underwear, the water-disintegrable resin film breaks in the thickness direction to thereby cause delamination. This delamination leads to the problem that the adhesive remains on the underwear and soils the underwear. For this reason, the content is preferably 1 to 50% by weight, more preferably 10 to 45% by weight.

Meanwhile, if the ethylene content in the ethylene/vinyl alcohol copolymer is large, the water-disintegradability of the water-disintegrable resin film to be obtained becomes lower to an extent that the disadvantages like those described above may occur when the water-disintegrable resin film is used as the back sheet of a liner for sanitary goods. In addition, a large ethylene content impairs the compatibility of the ethylene/vinyl alcohol copolymer with the oxyalkylene-modified polyvinyl alcohol-based resin and tends to make the forming into a water-disintegrable resin film unstable. Therefore, it is preferable that the ethylene content is 15 mol% or less. If the content is small, the water resistance of the water-disintegrable resin film to be obtained becomes lower to an extent that the disadvantages like those described above occur when the water-disintegrable resin film is used as the back sheet of the liner for sanitary goods. Therefore, it is more preferable that the content is 3 to 15 mol%.

As to the content of the ethylene/vinyl alcohol copolymer in the mixture of the oxyalkylene-modified polyvinyl alcohol-based resin and the ethylene/vinyl alcohol copolymer, if the content is small, the water resistance of the water-disintegrable resin film to be obtained becomes lower, whereas, if the content is large, the water-disintegradability of the water-disintegrable resin film to be obtained becomes lower to an extent that the respective disadvantages like those described above may occur when the water-disintegrable resin film is used as the back sheet of a liner for sanitary goods. For this reason, the content of the ethylene/vinyl alcohol copolymer is preferably 25 to 3200 parts by weight per 100 parts by weight of the oxyalkylene-modified polyvinyl alcohol-based resin.

Further, in order to facilitate the adjustment of the tensile strength and the start time for the disintegration of the water-disintegrable resin film by water to the values in the ranges described above, it is preferable that the ethylene/vinyl alcohol copolymer comprises of a combination of a low-polymerization-degree ethylene/vinyl alcohol copolymer having a lower degree of polymerization and a high-polymerization-degree ethylene/vinyl alcohol copolymer having a higher degree of polymerization.

If the degree of polymerization of the low-polymerization-degree ethylene/vinyl alcohol copolymer is small, the water resistance of the water-disintegrable resin film to be obtained becomes lower, whereas, if the degree of polymerization is large, the water-disintegradability of the water-disintegrable resin film to be obtained becomes lower to an extent that the respective disadvantages like those described above may occur when the water-disintegrable resin film is used as the back sheet of a liner for sanitary goods. For this reason, the degree of polymerization is preferably 300 to 600, more preferably 350 to 550.

As to the content of the low-polymerization-degree ethylene/vinyl alcohol copolymer in the ethylene/vinyl alcohol copolymer, if the content is small, the water-disintegradability of the water-disintegrable resin film to be obtained becomes lower, whereas, if the content is large, the water resistance of the water-disintegrable resin film to be obtained becomes lower to an extent that the respective disadvantages like those described above may occur when the water-disintegrable resin film is used as the back sheet of a liner for sanitary goods. For this reason, the content is preferably 30 to 95% by weight.

Meanwhile, if the degree of polymerization of the high-polymerization-degree ethylene/vinyl alcohol copolymer is small, the water resistance of the water-disintegrable resin film to be obtained becomes lower, whereas, if the degree of polymerization is large, the water-disintegradability of the water-disintegrable resin film to be obtained becomes lower to an extent that the respective disadvantages like those described above may occur when the water-disintegrable resin film is used as the back sheet of a liner for sanitary goods. For this reason, the degree of polymerization is preferably 700 to 2000, more preferably 800 to 1500.

As to the content of the high-polymerization-degree ethylene/vinyl alcohol copolymer in the ethylene/vinyl alcohol copolymer, if the content is small, the water resistance of the water-disintegrable resin film to be obtained becomes lower, whereas, if the content is large, the water-disintegradability of the water-disintegrable resin film to be obtained becomes lower to an extent that the respective disadvantages like those described above may occur when the water-disintegrable resin film is used as the back sheet of a liner for sanitary goods. For this reason, the content is preferably 5 to 70% by weight.

Further, if necessary, the polyvinyl alcohol-based resin may contain a plasticizer in order to improve film-forming property or the like. Examples of such plasticizers include water, glycerin, diglycerin, ethylene glycol, polyethylene glycol, polypropylene glycol and the like. Among these plasticizers, glycerin is preferable. These plasticizers may be used singly or in a combination of two or more.

If the amount of the plasticizer to be added is large, the bleeding out of the plasticizer from the water-disintegrable resin film to be obtained tends to occur and the change of physical properties of the water-disintegrable resin film with time becomes larger. For this reason, the amount is preferably 15 parts by weight or less, more preferably 10 parts by weight or less, per 100 parts by weight of the polyvinyl alcohol-based resin.

As to the thickness of the water-disintegrable resin film composed of the polyvinyl alcohol-based resin, if the thickness is small, the water resistance of the water-disintegrable resin film to be obtained becomes lower, whereas, if the thickness is large, the water-disintegradability of the water-disintegrable resin film to be obtained becomes lower to an extent that the respective disadvantages like those described above may occur when the water-disintegrable resin film is used as the back sheet of a liner for sanitary goods. For this reason, the thickness is preferably 10 to 100 µm, more preferably 15 to 50 µm.

The water-disintegrable resin film can be produced by a film-producing method hitherto generally employed. Examples of the method include a melt extrusion method, a solution casting method and the like.

Further, the water-disintegrable resin film may be a water-disintegrable resin film whose water resistance is improved by modifying one side or both sides thereof. Examples of such water-disintegrable resin films include ① a water-disintegrable resin film whose one side or both sides have undergone a thermal treatment to raise the degree of crystallization of the surface region in the one side or both sides of the water-disintegrable resin film so that the surface region is insolubilized; ② a water-disintegrable resin film obtained by carrying out the crosslinking of the water-disintegrable resin film by the addition thereto of a crosslinking agent; ③ a water-disintegrable resin film whose one side or both sides have been irradiated with an ionizing radiation to crosslink the surface region in the one side or both sides of the water-disintegrable resin film so that the surface region is insolubilized; ④ a water-disintegrable resin film whose one side or both sides have been brought into contact with a crosslinking agent to crosslink the surface region so that the surface region is insolubilized; and the like. Examples of the crosslinking agent include an aldehyde, a methylol compound, a vinyl compound, an epoxy compound, an ester compound, a metal compound and the like.

The degree of biodegradation after 28 days of the water-disintegrable resin film, which is constructed as described above, is preferably 30% or more, more preferably 40% or more in order to lessen the load to be placed on a sewage facility at the time when the water-disintegrable resin film is used as the back sheet of a liner for sanitary goods and the liner for sanitary goods is disposed in a sewage facility such as a sewage pipe and a septic tank. The degree of biodegradation means the value obtained by the measurement according to JIS K 6950.

Next, a laminated water-disintegrable resin film described in claim 4 will be described. The laminated water-disintegrable resin film is produced by integrally laminating a waterproofing layer on one side or both sides of a water-disintegrable resin film of 10 to 100 µm in thickness composed of a polyvinyl alcohol-based resin having a degree of saponification of 95 mol% and a degree of polymerization of 300 to 2000, and is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

That is, a waterproofing layer is integrally laminated on one side or both sides of the water-disintegrable resin film through an adhesive layer or directly without through the adhesive layer.

The description of the above-mentioned water-disintegrable resin film will not be given because the construction of this water-disintegrable resin film is the same as the construction of the water-disintegrable resin film described previously except that, whereas the synthetic resin constituting the above-mentioned water-disintegrable resin film is limited to a polyvinyl alcohol-based resin having a degree of saponification of 95 mol% or more and a degree of polymerization of 300 to 2000 and the thickness of the above-mentioned water-disintegrable resin film is limited to 10 to 100 µm, the tensile strength is not limited to 2 N/25 mm width or more and the start time for the disintegration of the water-disintegrable resin film by water is not limited to 600 seconds or less.

The synthetic resin constituting the waterproofing layer is not particularly limited in so far as the synthetic resin has a water resistance sufficient to prevent water from coming into contact with the water-disintegrable resin film from the waterproofing layer side. Examples of the synthetic resin include a thermoplastic polyurethane-based resin, a cellulose-based resin, polyhydroxy butyrate, polyhydroxy valerate, a copolymer of polyhydroxy butyrate and polyhydroxy valerate, starch, chitosan, polycaprolactam, polybutylene succinate or a modified product thereof, polyethylene succinate or a modified product thereof, polylactic acid, an aromatic polyester-based resin, an aliphatic polyester-based resin and the like. These resins may be used singly or in a combination of two or more.

Among the synthetic resins constituting the waterproofing layer, a thermoplastic polyurethane-based resin or a cellulose-based resin is preferable from the viewpoints of excellent water resistance, excellent biodegradability, and ease in adjusting the tensile strength and the start time for the disintegration of the water-disintegrable resin film by water to the values in the ranges that will be described later.

Preferred examples of the thermoplastic polyurethane-based resin include an ester-type resin, an ether-type resin and the like. An ester-type thermoplastic polyurethane-based resin is preferable because of excellence in biodegradability. The thermoplastic polyurethane-based resins may be used singly or in a combination of two or more.

Examples of the cellulose-based resins include ethyl cellulose, methyl cellulose, hydroxyethyl cellulose, hydroxypropyl cellulose, hydroxypropylmethyl cellulose, ethylhydroxyethyl cellulose, carboxymethyl cellulose, carboxymethylethyl cellulose, cellulose acetate and the like. Among these cellulose-based resins, ethyl cellulose is preferable because of its excellence in water resistance. These cellulose-based resins may be used singly or in a combination of two or more.

If necessary, a plasticizer may be added to the cellulose-based resin. In particular, in the case where the laminated water-disintegrable resin film is used as the back sheet of the liner for sanitary goods, an external force such as bending, shearing, stretching or the like is applied and flexibility is often required. For this reason, the addition of a plasticizer is preferable.

Examples of the plasticizer include a vegetable oil, an animal oil, an ester-based compound, an amide-based compound, a mineral oil, a fatty acid, an aliphatic alcohol and the like. These plasticizers may be used singly or in a combination of two or more.

More specifically, examples of the vegetable oil include castor oil, soybean oil, coconut oil, linseed oil, cotton seed oil, rapeseed oil, tung oil and the like. Examples of the animal oil include tallow, squalane, lanolin and the like. Examples of the ester-based compound include triethyl phosphate, tri-2-ethylhexyl phosphate, triphenyl phosphate, tricresyl phosphate, butylmethyl phthalate, butylcyclohexyl phthalate, diamyl phthalate, dibutyl phthalate, diphenyl phthalate, butyl oleate, diisooctyl adipate, triethyleneglycol-di-2-ethyl butyrate, butylphthalylbutyl glycolate, acetyltriethyl citrate, acetyltributyl citrate and the like. Examples of the amide-based compound include p-toluenesulfonic acid amide, ethyl-p-toluenesulfonic acid amide and the like.

If the amount of the plasticizer to be added is small, the effect of the addition is not obtained. On the other hand, if the amount of the plasticizer to be added is large, the mechanical strength of the laminated water-disintegrable resin film becomes lower, or the plasticizer tends to bleed out to the surface of the laminated water-disintegrable resin film and the change of physical properties of the laminated water-disintegrable resin film with time becomes larger. For this reason, the amount is preferably 0.1 to 70 parts by weight, more preferably 1 to 60 parts by weight, per 100 parts by weight of the cellulose-based resin.

As to the thickness of the waterproofing layer, it may be adjusted appropriately depending on the water-disintegradability and water resistance of the laminated water-disintegrable resin film to be obtained. If the thickness is small, the water resistance of the laminated water-disintegrable resin film to be obtained becomes lower, whereas, if the thickness is large, the water-disintegradability of the laminated water-disintegrable resin film to be obtained becomes lower and the respective disadvantages like those described above may occur when the laminated water-disintegrable resin film is used as the back sheet of a liner for sanitary goods. For this reason, the thickness is preferably 0.01 to 50 µm, more preferably 0.1 to 20 µm, and further more preferably 1 to 15 µm.

Besides, as to the water vapor permeability of the waterproofing layer, if it is large and the waterproofing layer side of the laminated water-disintegrable resin film to be obtained is made into a wet condition, the water vapor passes through the waterproofing layer and comes into contact with the water-disintegrable resin film. As a result, the mechanical strength of the laminated water-disintegrable resin film tends to become lower. For this reason, the water vapor permeability is preferably 7000 g/(m²·24 hours) or less, more preferably 4000 g/(m²·24 hours) or less.

The water vapor permeability is the value obtained by the measurement at a testing temperature of 40±0.5°C and a relative humidity difference (90±2)% according to Method A of JIS K 7129. The testing temperature is based on the assumption that, when the laminated water-disintegrable resin film to be obtained is used as the back sheet of a liner for sanitary goods, the temperature of the liner for sanitary goods becomes about 40°C by being steamed by body temperature in summer. The relative humidity difference is based on the assumption of sweat or the like from the body when the laminated water-disintegrable resin film is used as the back sheet of a liner for sanitary goods.

The waterproofing layer may be integrally laminated on one side or both sides of the water-disintegrable resin film through an adhesive layer. Examples of the adhesive resin constituting such an adhesive layer include an isocyanate-based resin, an polyethyleneimine-based resin, a modified polyolefin-based resin, a polyester-based resin, a polyurethane-based resin, a polybutadiene-based resin, a polyacrylamine-based resin, an oxazoline-based resin, an alkyl titanate-based resin and the like. These resins may be used singly or in a combination of two or more.

As to the thickness of the adhesive layer, if the thickness is small, the integrality between the water-disintegrable resin film and the waterproofing layer is impaired, whereas, if the thickness is large, the water-disintegradability of the laminated water-disintegrable resin film to be obtained is lowered and the disadvantages like those described above may occur when the laminated water-disintegrable resin film is used as the back sheet of a liner for sanitary goods. For this reason, the thickness is preferably 0.01 to 20 µm, more preferably 0.05 to 5 µm.

When the laminated water-disintegrable resin film is used as the back sheet of a liner for sanitary goods in a wet and warm or hot condition, the mechanical strength of the laminated water-disintegrable resin film is reduced. Further, fracture such as break occurs if a deforming stress is applied, or delamination occurs as the back sheet breaks in the thickness direction when the liner for sanitary goods is removed after use thereof from underwear. Accordingly, the tensile strength of the laminated water-disintegrable resin film is specified to 2 N/25 mm width or more. However, if the tensile strength is too large, the water-disintegradability drops to an extent that the start time for the disintegration of the laminated water-disintegrable resin film by water becomes longer and the disadvantages like those described above may occur when the laminated water-disintegrable resin film is used as the back sheet of the liner for sanitary goods. Accordingly, the tensile strength is preferably 2 to 30 N/25 mm width, more preferably 3 to 20 N/25 mm width. Besides, since the method for measuring the tensile strength of the laminated water-disintegrable resin film is the same as that the method for measuring the tensile strength of the water-disintegrable resin film, the description of the method for measuring the tensile strength of the laminated water-disintegrable resin film will not be given.

If the start time for the disintegration by water of the laminated water-disintegrable resin film is long and the laminated water-disintegrable resin film is used as the back sheet of a liner for sanitary goods and the liner for sanitary goods is disposed in a toilet, the liner for sanitary goods tends to choke the pipes of sewage or the liner for sanitary goods remains without being disintegrated in the sewage facility such as a sewage pipe and a septic tank for a long time thereby placing a large load on the sewage facility. For this reason, the time is specified as 600 seconds or less. On the other hand, if this time is short, even when the laminated water-disintegrable resin film is brought into contact with a small amount of water, the laminated water-disintegrable resin film tends to break. In addition, when the laminated water-disintegrable resin film is used as the back sheet of a liner for sanitary goods, the mechanical strength of the laminated water-disintegrable resin film is reduced particularly at a high temperature in summer when the liner for sanitary goods is in a wet and warm or hot condition due to perspiration from the body. In such a condition, if an external force is applied, for example, in a sitting condition, the laminated water-disintegrable resin film tends to break. For this reason, the start time for the disintegration by water of the laminated water-disintegrable resin film is preferably 20 to 600 seconds, more preferably 30 to 300 seconds, most preferably 40 to 180 seconds. Besides, since the method for measuring the start time for the disintegration by water of the laminated water-disintegrable resin film is the same as the method for measuring the start time for the disintegration by water of the water-disintegrable resin film, the description of the method for measuring the start time for the disintegration by water of the laminated water-disintegrable resin film will not be given.

If the tensile strength at 2% elongation of the laminated water-disintegrable resin film is small, the laminated water-disintegrable resin film loses its rigidity to an extent that the ease in handling is impaired. On the other hand, if this strength is large, the laminated water-disintegrable resin film imparts a hard feel to an extent that the suitability to wear is impaired when the laminated water-disintegrable resin film is used as the back sheet of a liner for sanitary goods, or the back sheet does not easily deform in compliance with the motion of the human body to an extent that the wearing of the liner for sanitary goods is uncomfortable. Accordingly, the tensile strength at 2% elongation is preferably 1 to 40 N/25 mm width, more preferably 2 to 30 N/25 mm width, most preferably 3 to 20 N/25 mm width. Besides, since the method for measuring the tensile strength at 2% elongation of the laminated water-disintegrable resin film is the same as the method for measuring the tensile strength at 2% elongation of the water-disintegrable resin film, the description of the method for measuring the tensile strength at 2% elongation of the laminated water-disintegrable resin film will not be given.

When the laminated water-disintegrable resin film having the above-described construction is used as the back sheet of a liner for sanitary goods or the like, the degree of biodegradation after 28 days is preferably 30% or more, more preferably 40% or more, in order to lessen the load to be placed on a sewage facility such as a sewage pipe or a septic tank in the case where the liner for sanitary goods is disposed in the sewage facility. The degree of biodegradation means the value obtained by the method according to JIS K 6950.

The method for integrally laminating the waterproofing layer on one side or both sides of the water-disintegrable resin film may be a method generally employed hitherto. Examples of the method include ① a method in which a polyvinyl alcohol-based resin constituting the water-disintegrable resin film and a synthetic resin constituting the waterproofing layer are coextruded; ② a method in which a polyvinyl alcohol-based resin constituting the water-disintegrable resin film and a synthetic resin constituting the waterproofing layer are formed separately into films and the two films are integrally laminated by means of heat press; ③ a method in which first a water-disintegrable resin film is produced and one side of the film is then coated with a synthetic resin constituting the waterproofing layer; and the like. It is often difficult to form a film constituting the waterproofing layer on its own because the waterproofing layer is a thin film. For this reason, it is preferable to employ the method in which first a water-disintegrable resin film is prepared and one side of the film is then coated with a synthetic resin constituting the waterproofing layer.

Examples of the method, in which first a water-disintegrable resin film is prepared and one side of the film is then coated with a synthetic resin constituting the waterproofing layer, include a coating method such as a gravure roll method, a kiss roll method, a knife coat method, a blade coat method, an air doctor coat method or the like, an extrusion lamination method, a casting method, a spraying method, a curtain method, a calendering method and the like. Among these methods, preferable is a roll coating method and more preferable is a gravure roll coating method because a waterproofing layer, which has a uniform thickness and is a thin film, can be formed on the surface of the water-disintegrable resin film.

Further, the method for integrally laminating a waterproofing layer on one side or both sides of the water-disintegrable resin film through the adhesive layer may be a method generally employed hitherto. Examples of the method include ① a method in which a polyvinyl alcohol-based resin constituting the water-disintegrable resin film, an adhesive resin constituting the adhesive layer, and a synthetic resin constituting the waterproofing layer are coextruded so that integral lamination in that order is carried out; and ② a method in which first a water-disintegrable resin film is produced and one side of the film is then coated with an adhesive resin constituting the adhesive layer either by direct coating or by transfer lamination of the adhesive resin that has been coated on an in-process paper in advance and the adhesive layer thus formed is coated with a synthetic resin constituting the waterproofing layer. Among these methods, preferable is the method ② because an adhesive layer and a waterproofing layer, each of which has a uniform thickness and is a thin film, can be formed on the surface of the water-disintegrable resin film.

Examples of the method for coating the adhesive resin constituting the adhesive layer on the surface of the water-disintegrable resin film or on the in-process paper include the same method as that for forming the waterproofing layer.

Besides, since the method for coating the synthetic resin constituting the waterproofing layer on the adhesive layer is the same as the method for coating the waterproofing layer on the surface of the water-disintegrable resin film, the description of the method for coating the synthetic resin constituting the waterproofing layer on the adhesive layer will be omitted.

Next, a water-disintegrable composite laminate which is described in claim 9 will be described. The water-disintegrable composite laminate is produced by integrally laminating a waterproofing layer on one side of a water-disintegrable resin film of 10 to 100 µm in thickness, composed of a polyvinyl alcohol-based resin having a degree of saponification of 95 mol% or more and a degree of polymerization of 300 to 2000, through an adhesive layer or without through the adhesive layer, and integrally laminating a water-disintegrable backup material on the other side thereof, and is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

Besides, since the water-disintegrable composite laminate has the same construction as that of the laminated water-disintegrable resin film described in claim 4 except that one side thereof has a water-disintegrable backup material integrally laminated thereon, the description of the same part will not be given.

One side of the water-disintegrable resin film in the water-disintegrable composite laminate has a water-disintegrable backup material integrally laminated thereon. This construction is designed to prevent the water-disintegrable composite laminate, which gets separated from a liquid absorbent or the top sheet, from curling in a state of a cigar (i.e., a spiral) with one side thereof facing inwardly such that film surfaces adheres to each other to an extent that the water-disintegradability of the water-disintegrable composite laminate become lower in the case where the water-disintegrable composite laminate is used as the back sheet of a liner for sanitary goods and the liner for sanitary goods is disposed after use in a sewage facility.

The water-disintegrable backup material may be integrally laminated on the water-disintegrable resin film though the waterproofing layer.

The water-disintegrable backup material is not particularly limited in so far as its shape is gradually lost as it absorbs water. A preferred example of the water-disintegrable backup material is a nonwoven fabric or nonwoven sheet comprising biodegradable fibers bound to each other by means of a water-soluble binder. Examples of the biodegradable fibers include pulp, rayon, cupra, cotton, flax, wool, silk and the like. Among these fibers, pulp is preferable.

Examples of the water-soluble binder include a polyvinyl alcohol-based resin, a cellulose-based resin such as methyl cellulose or hydroxyethyl cellulose, starch, sodium alginate, sodium polyacrylate, polyacrylic ester and the like.

If the tensile strength of the water-disintegrable composite laminate is small, when the water-disintegrable composite laminate is used as the back sheet of a liner for sanitary goods in a wet and warm or hot condition, the mechanical strength of the water-disintegrable composite laminate is reduced. If a deforming stress is applied, fracture such as break occurs or delamination occurs as the back sheet breaks in the thickness direction when the liner for sanitary goods is removed after use thereof from underwear. Accordingly, the tensile strength is specified as 2 N/25 mm width or more. On the other hand, the upper limit of the tensile strength is not particularly specified by a value. The larger the tensile strength of the water-disintegrable composite laminate, the more desirable the film is. However, if this value of the water-disintegrable composite laminate is larger, the film has a larger rigidity and therefore does not satisfy the suitability to wear. Accordingly, the tensile strength is preferably 2 to 30 N/25 mm width and a more preferable range is 3 to 20 N/25 mm width. Besides, since the method for measuring the tensile strength of the water-disintegrable composite laminate is the same as the method for measuring the tensile strength of the water-disintegrable resin film, the description of the method for measuring the tensile strength of the water-disintegrable composite laminate will not be given.

If the start time for the disintegration by water of the water-disintegrable composite laminate is long, when the water-disintegrable composite laminate is used as the back sheet of a liner for sanitary goods and the liner for sanitary goods is disposed in a toilet, the liner for sanitary goods tends to choke the pipe of sewage or the liner for sanitary goods remains without being water-disintegrated in the sewage facility such as a sewage pipe and a septic tank for a long time thereby placing a large load on the sewage facility. For this reason, the time is specified as 600 seconds or less. On the other hand, if this time is short, even when the water-disintegrable composite laminate is brought into contact with a small amount of water, the water-disintegrable composite laminate tends to break. In addition, in the case where the water-disintegrable composite laminate is used as the back sheet of the liner for sanitary goods, the mechanical strength of the water-disintegrable composite laminate is reduced particularly at a high temperature in summer when the liner for sanitary goods is in a wet and warm or hot condition due to perspiration from the body. In such a condition, if an external force is applied, for example, in a sitting condition, the water-disintegrable composite laminate tends to break. For this reason, the start time for the disintegration of the water-disintegrable composite laminate by water is preferably 20 to 600 seconds, more preferably 30 to 300 seconds, most preferably 40 to 180 seconds. Besides, since the method for measuring the start time for the disintegration of the water-disintegrable composite laminate by water is the same as the method for measuring the start time for the disintegration of the water-disintegrable resin film by water, the description of the method for measuring the start time for the disintegration of the water-disintegrable composite laminate by water will not be given.

The degree of biodegradation after 28 days of the water-disintegrable composite laminate, which is constructed as described above, is preferably 30% or more, more preferably 40% or more, in order to lessen the load to be placed on sewage facility at the time when the water-disintegrable composite laminate is used as the back sheet of a liner for sanitary goods and the liner for sanitary goods is disposed in a sewage facility such as a sewage pipe and a septic tank. The degree of biodegradation means the value obtained by the measurement according to JIS K 6950.

If the tensile strength at 2% elongation of the water-disintegrable composite laminate is small, the water-disintegrable composite laminate loses its rigidity to an extent that the ease in handling is impaired. On the other hand, if this strength is large, the water-disintegrable composite laminate imparts a hard feel to an extent that the feeling of wear is impaired when the water-disintegrable composite laminate is used as the back sheet of a liner for sanitary goods, or the back sheet does not easily deform in compliance with the motion of the human body to an extent that the wearing of the liner for sanitary goods is uncomfortable. Accordingly, the tensile strength is preferably 1 to 40 N/25 mm width, more preferably 2 to 30 N/25 mm width, most preferably 3 to 20 N/25 mm width. Besides, since the method for measuring the tensile strength at 2% elongation of the water-disintegrable composite laminate is the same as the method for measuring the tensile strength at 2% elongation of the water-disintegrable resin film, the description of the method for measuring the tensile strength at 2% elongation of the water-disintegrable composite laminate will not be given.

The method for integrally laminating the water-disintegrable backup material on one side of the water-disintegrable resin film is not particularly limited. For example, a synthetic resin constituting the water-disintegrable resin film is extruded and laminated onto one side of the water-disintegrable backup material. The method for integrally laminating the water-disintegrable backup material on one side of the water-disintegrable resin film may be determined appropriately in accordance with the construction of the water-disintegrable composite laminate.

Next, a method for producing a water-disintegrable composite laminate will be described. As an example, the water-disintegrable composite laminate is produced by integrally laminating a waterproofing layer on one side of the water-disintegrable resin film through an adhesive layer and also integrally laminating a water-disintegrable backup material on the other side thereof. An example of producing this water-disintegrable composite laminate is as follows. First, the above-mentioned polyvinyl alcohol-based resin having a degree of saponification of 95 mol% or more and a degree of polymerization of 300 to 2000 is extruded and laminated at a thickness of 10 to 100 µm onto one side of a water-disintegrable backup material so that the water-disintegrable resin film is integrally laminated. After that, the surface of the water-disintegrable resin film is directly coated with an adhesive resin constituting the adhesive layer either by direct coating or by transfer lamination of the adhesive resin that has been coated on an in-process paper in advance and the adhesive layer thus formed is coated with a synthetic resin constituting the waterproofing layer.

As another example, the water-disintegrable composite laminate is produced by integrally laminating a waterproofing layer on one side of the water-disintegrable resin film through an adhesive layer and also integrally laminating a water-disintegrable backup material on the other side thereof through a waterproofing layer. An example of making this water-disintegrable composite laminate is as follows. First, the waterproofing synthetic resin is extruded and laminated onto the entire surface of one side of a water-disintegrable backup material so that a waterproofing layer is integrally laminated. Next, a polyvinyl alcohol-based resin having a degree of saponification of 95 mol% or more and a degree of polymerization of 300 to 2000 is extruded and laminated at a thickness of 10 to 100 µm onto the waterproofing layer so that the water-disintegrable resin film is integrally laminated. After that, an adhesive layer is integrally laminated on the water-disintegrable resin film and, thereafter, the waterproofing layer is integrally laminated on the adhesive layer.

Besides, in the case where the adhesive layer is not formed in the water-disintegrable composite laminate described above, the step in which the adhesive resin is coated on the water-disintegrable resin film will be omitted.

Next, manners, in which the water-disintegrable resin film, the laminated water-disintegrable resin film or the water-disintegrable composite laminate is used as the back sheet of a liner for sanitary goods, will be described. Since these manners are the same, the following description is limited to Fig. 3 illustrating a water-disintegrable composite laminate 6 produced by integrally laminating the waterproofing layer 63 on one side of the water-disintegrable resin film 61 through the adhesive layer 62 and also integrally laminating the water-disintegrable backup material 64 on the other side thereof. The description of a laminated water-disintegrable resin film and the water-disintegrable resin film, which do not have the water-disintegrable backup material 64 integrally laminated thereon, will not be given.

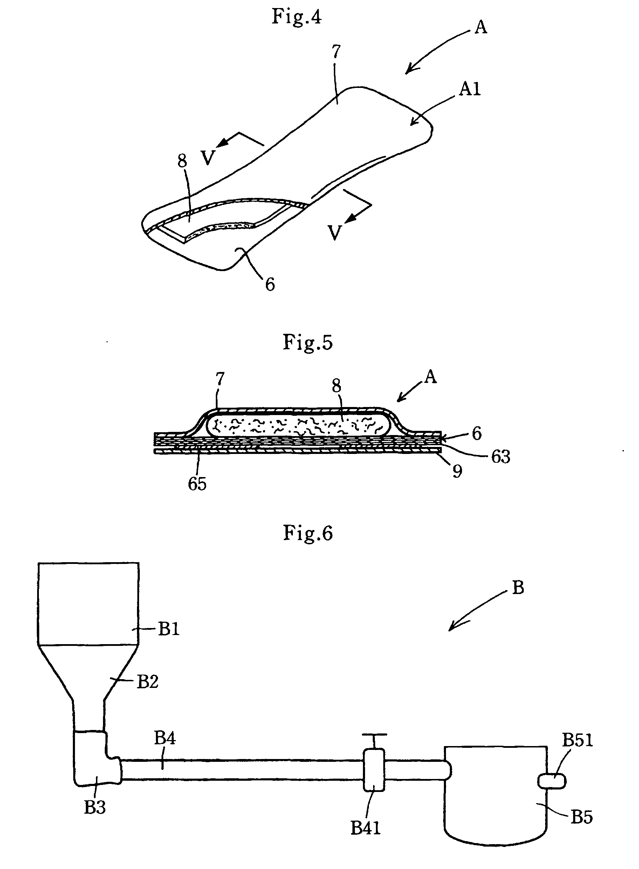

As illustrated in Figs. 4 and 5, a liner for sanitary goods A, which uses the water-disintegrable composite laminate 6 as the back sheet, comprises a liquid-permeable top sheet 7, which is permeable to such liquids as body fluids, blood, and urine and has a planar approximately rectangular shape having a larger length and a width slightly narrowing gradually towards the central part, and the water-disintegrable composite laminate 6, which has the same shape and the same size as those of the liquid-permeable top sheet 7, wherein the liquid-permeable top sheet 7 and the water-disintegrable composite laminate 6 are overlapped with each other in such a manner that the waterproofing layer 63 of the water-disintegrable composite laminate 6 faces outward and the opposing outer peripheries of the liquid-permeable top sheet 7 and the water-disintegrable composite laminate 6 are integrated by means of thermal fusion, adhesive or the like to thereby form a container A1 and a liquid absorbent 8 is put in the container A1 which is formed by the space between the opposing inner faces of the liquid-permeable top sheet 7 and the water-disintegrable composite laminate 6. The adhesive layer 65 for fixing the liner for sanitary goods A to underwear is formed in a suitable state such as dots, stripes and beads on the surface of the liner for sanitary goods A on the waterproofing layer 63 side and the release paper 9 is peelably laminated on the surface of the adhesive layer.

The liquid-permeable top sheet 7 is not particularly limited in so far as it is one used hitherto as the top sheet of the liner for sanitary goods. Taking into consideration that a liner for sanitary goods after use is disposed in a sewage facility, it is preferable that the liquid-permeable top sheet 7 is a fibrous article such as a nonwoven fabric which is soluble in water or gradually loses the shape by the disentanglement of the fibers so that the fibers separate.

The liquid absorbent 8 is not particularly limited in so far as it is one used hitherto as the liquid absorbent of a liner for sanitary goods. Taking into consideration that the liner for sanitary goods after use is disposed in a sewage facility, it is preferable that the liquid absorbent 8 is a fibrous article such as a thick nonwoven fabric which is soluble in water or gradually loses the shape by the disentanglement of the fibers so that the fibers separate.

Examples of the fibers that are used in the liquid-permeable top sheet 7 and the liquid absorbent 8 include pulp, rayon, cupra, cotton, flax, wool, silk and the like. Among these fibers, fibers that are biodegradable are suitably used.

The liner for sanitary goods A, which has the construction described above, is used in a state fixed to the underwear by the adhesive layer 65 of the water-disintegrable composite laminate 6 (back sheet). However, since the tensile strength of the water-disintegrable composite laminate 6 used in the liner for sanitary goods A is specified as 2 N/25 mm width or more, the water-disintegrable composite laminate 6 maintains the predetermined mechanical strength even when the water-disintegrable composite laminate 6 is in a wet and warm or hot condition due to the body temperature and the sweat from the body. Accordingly, even in the case where an external force such as a pressing force is applied to the liner for sanitary goods A by, for example, the motion or sitting of the user, the water-disintegrable composite laminate 6 that is the back sheet does not break and can hold the liquid absorbent 8 inside the container A1 in a stable and reliable manner. As a result, the case, where the liquid absorbed in the liquid absorbent 8 leaks unexpectedly out of the liner for sanitary goods A, can be prevented without fail.

Even if the liner for sanitary goods A is disposed in a sewage facility after using, the liner for sanitary goods A absorbs the water in the sewage facility and is rapidly water-disintegrated because the water-disintegrable composite laminate is characterized by the start time for the disintegration of the water-disintegrable composite laminate by water of 600 seconds or less. Therefore, it does not happen that the liner for sanitary goods A remains in the sewage facility for a long period of time and places a load on the sewage facility.

### BRIEF DESCRIPTION OF THE DRAWINGS

Fig. 1 is a partially notched schematic perspective view showing a measuring manner of a tensile strength of a water-disintegrable resin film, Fig. 2 is a cross-sectional view taken along line II-II of Fig. 1, Fig. 3 is a cross-sectional view of a laminated water-disintegrable resin film, Fig. 4 is a partially notched perspective view showing one example of a liner for sanitary goods using a water-disintegrable composite laminate, Fig. 5 is a cross-sectional view taken along line V-V of the liner for sanitary goods of Fig. 4, and Fig. 6 is a schematic side view of an apparatus used for measuring the disposability of the water-disintegrable resin film (laminated water-disintegrable resin film, water-disintegrable composite laminate).

### BEST MODES FOR CARRYING OUT THE INVENTION

### (Examples 1 to 3)

Polyvinyl alcohol-based resins made of predetermined amounts, shown in Table 1, of an oxyalkylene-modified polyvinyl alcohol (polyoxyethylene group content: approximately 23% by weight, degree of saponification: 99 mol%, degree of polymerization: 500), an ethylene/vinyl alcohol copolymer (1) (ethylene content: 6 mol%, degree of saponification: 99 mol%, degree of polymerization: 500) and an ethylene/vinyl alcohol copolymer (2) (ethylene content: 6 mol%, degree of saponification: 99 mol%, degree of polymerization: 1000) as well as glycerin as a plasticizer were fed to an extruder, molten and blended in the extruder and, then, extruded from a T-shaped die. Thus, a water-disintegrable resin film of 20 µm in thickness was obtained.

Then, the entire surface of one side of the water-disintegrable resin film was coated with an ethyl cellulose (trade name "N-100" manufactured by Hercules Incorporated) solution in a toluene/methanol mixed solvent by a gravure roll method to thereby integrally laminate a waterproofing layer of 4 µm in thickness on the water-disintegrable resin film. Thus, a laminated water-disintegrable resin film was obtained.

### (Example 4)

A laminated water-disintegrable resin film was obtained in the same manner as that in Example 3, except that the water-disintegrable resin film had a thickness of 30 µm.

### (Example 5)

A laminated water-disintegrable resin film was obtained in the same manner as that in Example 1, except that one side of the water-disintegrable resin film was coated with an ester-type thermoplastic polyurethane-based resin (trade name "Pandex T-5205" manufactured by Dainippon Ink & Chemicals, Inc.) in place of ethyl cellulose to thereby integrally laminate a waterproofing layer of 5 µm in thickness on the water-disintegrable resin film.

### (Example 6)

The entire surface of one side of the water-disintegrable resin film obtained in the same manner as that in Example 1 was coated with a urethane-based adhesive by a gravure roll method to thereby integrally laminate an adhesive layer of 1 µm in thickness on the water-disintegrable resin film. After that, the entire surface of the adhesive layer was coated with the same ester-type thermoplastic polyurethane-based resin as that in Example 5 by a gravure roll method to thereby integrally laminate a waterproofing layer of 5 µm in thickness on the water-disintegrable resin film. Thus, a laminated water-disintegrable resin film was obtained.

### (Examples 7, 8)

Polyvinyl alcohol-based resins made of predetermined amounts, shown in Table 1, of an oxyalkylene-modified polyvinyl alcohol, an ethylene/vinyl alcohol copolymer (1) and an ethylene/vinyl alcohol copolymer (2) as well as glycerin as a plasticizer were fed to an extruder, molten and blended in the extruder and, then, extruded and laminated at a thickness of 20 µm onto the entire surface of one side of water-disintegrable paper (pulp 100%, basis weight: 20 g/m²) as a water-disintegrable backup material. Thus, a laminate having a water-disintegrable resin film laminated thereon was obtained.

Further, the entire surface of the water-disintegrable resin film was coated with a urethane-based adhesive by a gravure roll method to thereby integrally laminate an adhesive layer of 1 µm in thickness on the water-disintegrable resin film. After that, the entire surface of the adhesive layer was coated with the same ester-type thermoplastic polyurethane-based resin as that in Example 5 by a gravure roll method to thereby integrally laminate a waterproofing layer of 5 µm in thickness on the water-disintegrable resin film. Thus, a water-disintegrable composite laminate was obtained.

### (Example 9)

A water-disintegrable composite laminate was obtained in the same manner as that in Example 7, except that the water-disintegrable resin film, which was integrally laminated on one side of the water-disintegrable paper, had a thickness of 30 µm.

### (Example 10)

A laminated water-disintegrable resin film was obtained in the same manner as that in Example 8, except that the waterproofing layer, which was integrally laminated on the adhesive layer, had a thickness of 8 µm.

### (Example 11)

Polyvinyl alcohol-based resins made of predetermined amounts, shown in Table 1, of an oxyalkylene-modified polyvinyl alcohol, an ethylene/vinyl alcohol copolymer (1) and an ethylene/vinyl alcohol copolymer (2) as well as glycerin as a plasticizer were fed to an extruder, molten and blended in the extruder and, then, extruded from a T-shaped die. Thus, a water-disintegrable resin film of 15 µm in thickness was obtained.

Further, the entire surface of the water-disintegrable resin film was coated with a urethane-based adhesive by a gravure roll method to thereby integrally laminate an adhesive layer of 1 µm in thickness on the water-disintegrable resin film. After that, the entire surface of the adhesive layer was coated with the same ester-type thermoplastic polyurethane-based resin as that in Example 5 by a gravure roll method to thereby integrally laminate a waterproofing layer of 5 µm in thickness on the water-disintegrable resin film. Thus, a laminated water-disintegrable resin film was obtained.

### (Example 12)

Polyvinyl alcohol-based resins made of predetermined amounts, shown in Table 1, of an oxyalkylene-modified polyvinyl alcohol, an ethylene/vinyl alcohol copolymer (1) and an ethylene/vinyl alcohol copolymer (2) as well as glycerin as a plasticizer were fed to an extruder, molten and blended in the extruder and, then, extruded and laminated at a thickness of 35 µm onto the entire surface of one side of water-disintegrable paper (pulp 100%, basis weight: 20 g/m²) as a water-disintegrable backup material. Thus, a laminate having a water-disintegrable resin film laminated thereon was obtained.

Further, the entire surface of the water-disintegrable, resin film was coated with a urethane-based adhesive by a gravure roll method to thereby integrally laminate an adhesive layer of 2 µm in thickness on the water-disintegrable resin film. After that, the entire surface of the adhesive layer was coated with the same ethyl cellulose as that in Example 1 by a gravure roll method to thereby integrally laminate a waterproofing layer of 6 µm in thickness on the water-disintegrable resin film. Thus, a water-disintegrable composite laminate was obtained.

### (Comparative Example 1)

As shown in Table 1, 100 parts by weight of an oxyalkylene-modified polyvinyl alcohol and 8 parts by weight of glycerin as a plasticizer were fed to an extruder, molten and blended in the extruder and, then, extruded from a T-shaped die. Thus, a water-disintegrable resin film of 20 µm in thickness was obtained.

Then, the entire surface of one side of the water-disintegrable resin film was coated with the same ethyl cellulose as that in Example 1 by a gravure roll method to thereby integrally laminate a waterproofing layer of 2 µm in thickness on the water-disintegrable resin film. Thus, a laminated water-disintegrable resin film was obtained.

### (Comparative Example 2)

As shown in Table 1, 100 parts by weight of an ethylene/vinyl alcohol copolymer (3) (ethylene content: 32 mol%, degree of saponification: 99 mol%, degree of polymerization: 500) and 8 parts by weight of glycerin as a plasticizer were fed to an extruder, molten and blended in the extruder and, then, extruded from a T-shaped die. Thus, a water-disintegrable resin film of 20 µm in thickness was obtained.

Then, the entire surface of one side of the water-disintegrable resin film was coated with the same ethyl cellulose as that in Example 1 by a gravure roll method to thereby integrally laminate a waterproofing layer of 4 µm in thickness on the water-disintegrable resin film. Thus, a laminated water-disintegrable resin film was obtained.

### (Comparative Example 3)

As shown in Table 1, an oxyalkylene-modified polyvinyl alcohol and 8 parts by weight of glycerin as a plasticizer were fed to an extruder, molten and blended in the extruder and, then, extruded from a T-shaped die. Thus, a water-disintegrable resin film of 20 µm in thickness was obtained.

### (Comparative Example 4)

As shown in Table 1, 100 parts by weight of an ethylene/vinyl alcohol copolymer (3) and 8 parts by weight of glycerin as a plasticizer were fed to an extruder, molten and blended in the extruder and, then, extruded from a T-shaped die. Thus, a water-disintegrable resin film of 20 µm in thickness was obtained.

The water-disintegrable resin films, the laminated water-disintegrable resin films and the water-disintegrable composite laminates obtained in the above-described manners were subjected to the measurements of tensile strength, start time for the disintegration by water thereof, tensile strength at 2% elongation, and biodegradability after 28 days. In addition, the water vapor permeability of the waterproofing layer alone of each of the laminated water-disintegrable resin films and the water-disintegrable composite laminates was measured at the same thickness as the thickness of the waterproofing layer integrally laminated on the water-disintegrable resin film. Further, suitability to wear and disposability were measured according to the following methods. The results are shown in Table 1.

### (Suitability to wear)

A liquid-permeable top sheet which was in a planar approximately rectangular shape having a width of 55 mm × a length of 140 mm and made of a water-disintegrable pulp nonwoven fabric (basis weight: 40 g/m²), and a back sheet which had the same shape and the same size as those of the liquid-permeable top sheet and was composed of a water-disintegrable resin film, a laminated water-disintegrable resin film or a water-disintegrable composite laminate were overlapped with each other in such a manner that a liquid absorbent (aerated pulp, basis weight: 60 g/m²) was interposed between the opposing faces, and the opposing outer peripheries of the liquid-permeable top sheet and the back sheet were integrated by means of needle punching. Thus, a container was formed. Further, a belt-like adhesive layer (based on an acrylic resin) in a shape of a strip having a width of 25 mm and a length of 140 mm was laminated at a layer thickness of 5 µm in the length direction of the container on the central part of the outer face of the back sheet in the width direction thereof to thereby obtain a liner for sanitary goods. In the case where the laminated water-disintegrable resin film or the water-disintegrable composite laminate was used, the laminated water-disintegrable resin film or the water-disintegrable composite laminate was overlapped with the liquid-permeable top sheet in such a manner that the waterproofing layer faced outward.

The liner for sanitary goods was detachably fixed to underwear by means of the adhesive layer formed on the outer face of the back sheet of the liner for sanitary goods. Persons wearing the underwear were allowed to walk for 4 hours and to have a seat for 4 hours. After they put the underwear to the body for a total of 8 hours, the liner for sanitary goods was peeled from the underwear.

After that, the suitability to wear of the liner for sanitary goods was assessed based on the following criteria. The results are shown in Table 1.
ⓞ ··· There was no uncomfortable feeling during use and the feeling of use was good. After the use, the liner for sanitary goods could be peeled from the underwear without causing delamination.
○₁ ··· There was no uncomfortable feeling during use, but the handleability was poor when the liner for sanitary goods was used.
○₂ ··· There was some uncomfortable feeling during use, but the uncomfortable feeling did not cause any problem in the use of the liner for sanitary goods.
Δ ··· After use, when the liner for sanitary goods was peeled from the underwear, delamination (residue of adhesive) partially occurred, but there was no problem because the amount of the remaining adhesive was slight and the adhesive could be easily removed from the underwear.
× ··· After use, when the liner for sanitary goods was peeled from the underwear, delamination occurred in the entire face, and it was difficult to remove the adhesive from the underwear.

### (Disposability)

In an apparatus B which was used to measure the disposability, as shown in Fig. 6, the lower end of a cylindrical wastewater tank B1 which has a diameter of 40 cm and a height of 15 cm and is connected to and communicates with one end of a horizontal drain pipe B4 having an inner diameter of 75 mm and a length of 3 m via a funnel-shaped connection part B2 and a pipe joint B3 and, also, the other end of the drain pipe B4 is connected to and communicates with the upper peripheral end of a cylindrical pseudo septic tank B5 which has a diameter of 60 cm and a height of 60 cm, so that the wastewater tank B1 is connected to and communicates with the septic tank B5 via the drain pipe B4. The drain pipe B4 is equipped with a valve B41 for opening and closing the drain pipe B4. On the other hand, the upper peripheral end of the septic tank B5 is provided with an exhaust port B51 which opens from inside to outside of the wall.

The valve B41 of the drain pipe B4 in the apparatus B is closed and water is stored up to a depth of 5 cm in the wastewater tank B1. After that, the liner for sanitary goods used in the assessment of the suitability to wear is placed and retained in the water for 3 minutes.

Subsequently, the valve B41 of the drain pipe B4 is opened and, simultaneously, water is poured into the wastewater tank B1 at a flow rate of 40 liters/minute for 10 seconds. After that, the valve is closed. The opening degree of the valve is adjusted so that water is stored up to a depth of 5 cm in the wastewater tank B1 when the valve is closed. An excess of water that flows into the septic tank B5 is appropriately discharged from exhaust port B51.

In addition, a series of operations was carried out 12 times per day at a suitable interval of time, wherein the operations comprise opening the valve B41 of the drain pipe B4 and, simultaneously, pouring water into the wastewater tank B1 at a flow rate of 40 liters/minute for 10 seconds, and thereafter closing the valve. This series of operations was defined as one cycle and 31 cycles were repeated.

After 10 cycles and after 31 cycles, the liner for sanitary goods inside the septic tank B5 was visually inspected and the state was assessed based on the following criteria. The results are shown in Table 1.
ⓞ ··· After 10 cycles, the back sheet of the liner for sanitary goods was disintegrated by water to an extent that the original shape could not be confirmed.
○ ··· After 10 cycles, the original shape of the back sheet of the liner for sanitary goods could be confirmed. But after 31 cycles, the back sheet of the liner for sanitary goods was disintegrated by water to an extent that the original shape could not be confirmed.
× ··· After 31 cycles, the original shape of the back sheet in the liner for sanitary goods could be confirmed.

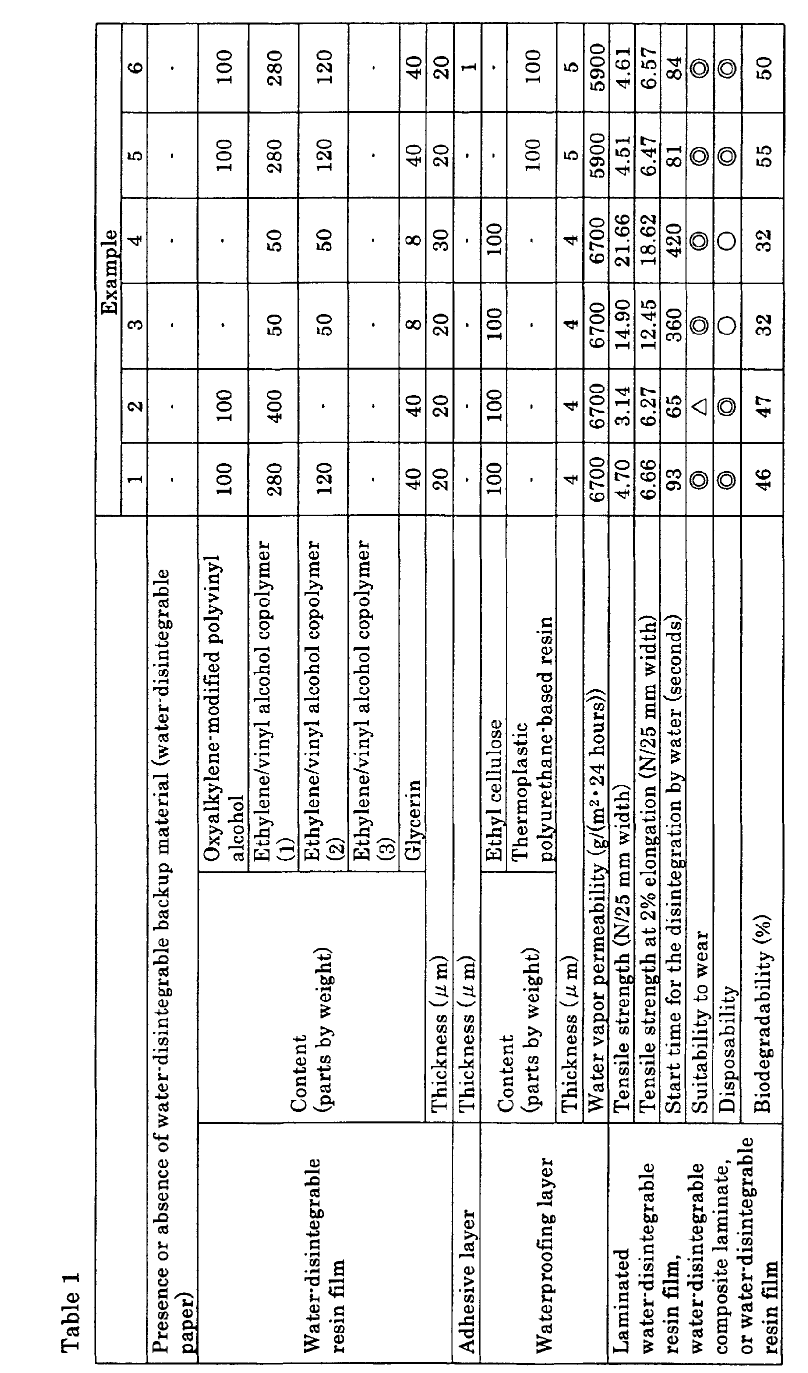

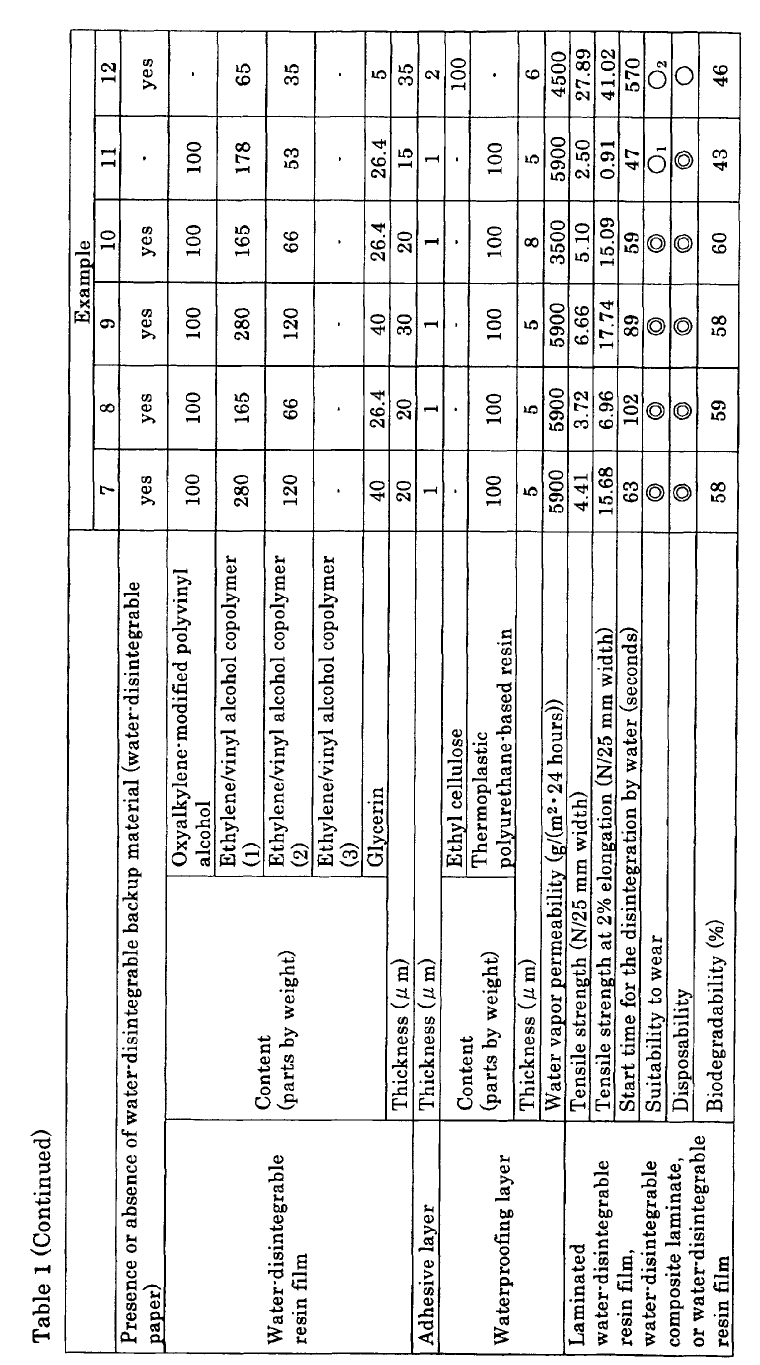

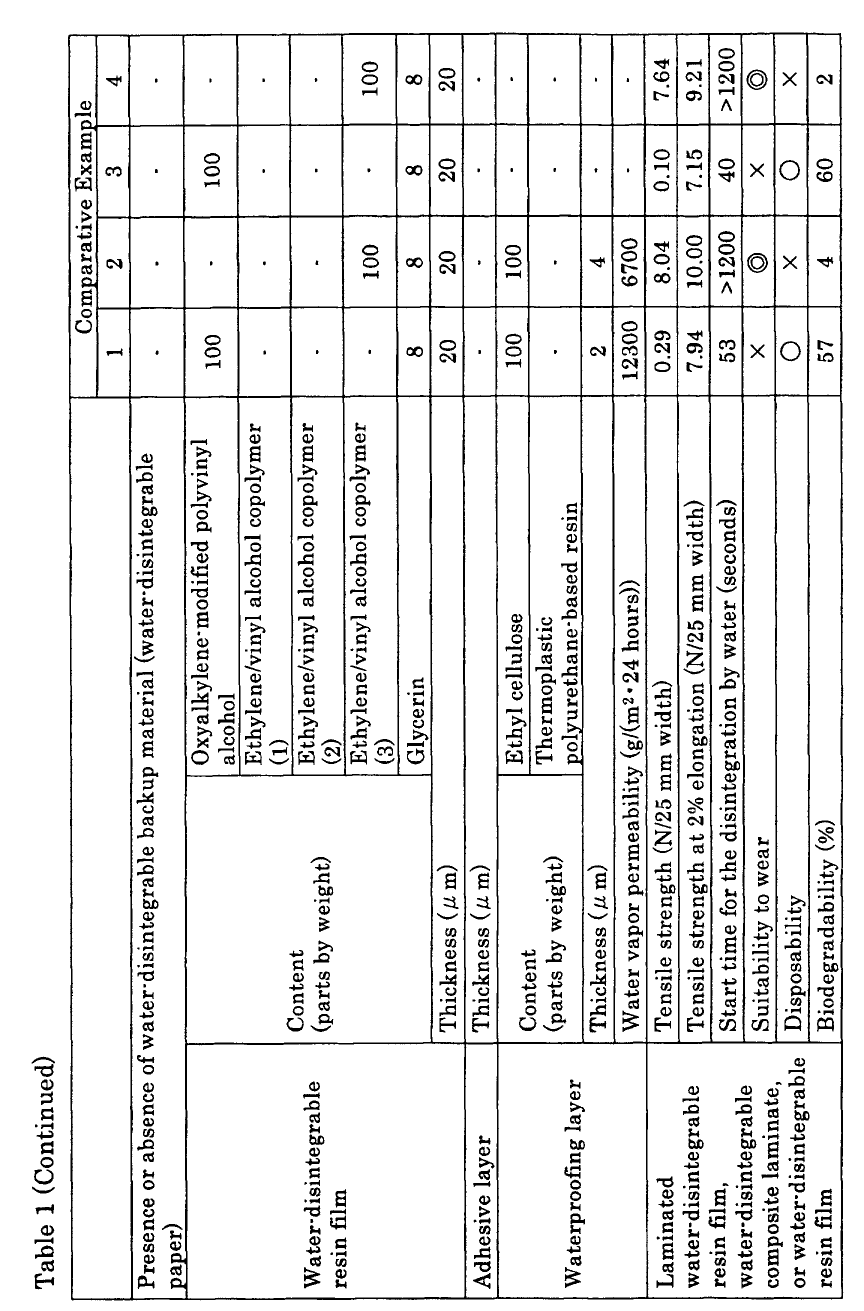

According to the present invention, a water-disintegrable resin film is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united. Therefore, the water-disintegrable resin film has a predetermined mechanical strength under a wet and warm or hot condition and also exhibits excellent water-disintegradability for a large amount of water, and thus can be suitably used as a back sheet of a liner for sanitary goods, which requires the combination of water resistance and good water-disintegradability.

The liner for sanitary goods is exposed for a long period of time to an environment in a wet and warm or hot condition due to the heat and sweat from the body during its use. However, as described above, since the water-disintegrable resin film has an excellent mechanical strength and an excellent water-disintegradability under a wet and warm or hot condition, the liner for sanitary goods using the water-disintegrable resin film as the back sheet does not undergo fracture such as break even if a deforming stress is applied from exterior by the motion such as sitting of the person who wears the liner for sanitary goods. Therefore, the liner for sanitary goods does not unexpectedly leak the liquids absorbed in the liquid absorbent in the liner for sanitary goods to the outside and, when the liner for sanitary goods after use is put in a sewage facility, the liner for sanitary goods undergoes rapid water-disintegradability and can be disposed in a simple and reliable manner without placing load on the sewage facility.

If the tensile strength at 2% elongation of the water-disintegrable resin film is 1 to 40 N/25 mm width, when a elongation stress is applied to the water-disintegrable resin film, the water-disintegrable resin film is elongated in compliance with the elongation stress in a smooth and reliable manner without being broken.

Meanwhile, although an elongation stress is applied to the liner for sanitary goods according to the motion of the person who wears the liner for sanitary goods, the use of the water-disintegrable resin film as the back sheet leads to the liner for sanitary goods excellent in suitability to wear because the water-disintegrable resin film is elongated smoothly in compliance with the elongation stress applied thereto without being broken.

Further, if the water-disintegrable resin film is a film of 10 to 100 µm in thickness, composed of a polyvinyl alcohol-based resin having a degree of saponification of 95 mol% or more and a degree of polymerization of 300 to 2000, the water-disintegrable resin film has a higher mechanical strength in a wet and warm or hot condition and has a better water-disintegradability. Therefore, the water-disintegrable resin film can be used more suitably as the back sheet of a liner for sanitary goods, which requires the combination of water resistance and water-disintegradability.

According to the present invention, a laminated water-disintegrable resin film is produced by integrally laminating a waterproofing layer on one side or both sides of a water-disintegrable resin film of 10 to 100 µm in thickness composed of a polyvinyl alcohol-based resin having a degree of saponification of 95 mol% and a degree of polymerization of 300 to 2000, and is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united. Therefore, the laminated water-disintegrable resin film has a predetermined mechanical strength under a wet and warm or hot condition and also exhibits excellent water-disintegradability for a large amount of water, and thus can be suitably used as a back sheet of a liner for sanitary goods, which requires the combination of water resistance and good water-disintegradability.

The polyvinyl alcohol-based resin constituting the water-disintegrable resin film of the laminated water-disintegrable resin film is composed of a predetermined oxyalkylene-modified polyvinyl alcohol-based resin and a predetermined ethylene/vinyl alcohol copolymer. Therefore, it is possible to obtain a laminated water-disintegrable resin film having a balance between mechanical strength under a wet and warm or hot condition and water-disintegradability.

If the waterproofing layer is composed of a thermoplastic polyurethane-based resin or a cellulose-based resin, it is possible to obtain a laminated water-disintegrable resin film having a better water-disintegradability.

Further, if the waterproofing layer is integrally laminated on the surface of the water-disintegrable resin film through an adhesive layer, the water-disintegrable resin film and the waterproofing layer can be tightly, integrally laminated. Therefore, even if a deforming stress is applied to the laminated water-disintegrable resin film, the water-disintegrable resin film does not peel from the waterproofing layer and the laminated water-disintegrable resin film maintains excellent tensile strength in a wet and warm or hot condition and water-disintegradability in a reliable manner.

Next, according to the present invention, a water-disintegrable composite laminate is produced by integrally laminating a waterproofing layer on one side of a water-disintegrable resin film of 10 to 100 µm in thickness, composed of a polyvinyl alcohol-based resin having a degree of saponification of 95 mol% or more and a degree of polymerization of 300 to 2000, through an adhesive layer or without through the adhesive layer, and integrally laminating a water-disintegrable backup material on the other side thereof, and is characterized in that it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united. When the water-disintegrable composite laminate is immersed in water, the above-described characteristics prevent the water-disintegrable backup material from curling the water-disintegrable composite laminate in a state of a cigar (i.e., a spiral) such that film surfaces adheres to each other and cause the water-disintegrable backup material to absorb water positively so that the water absorption of the water-disintegrable composite laminate as a whole is accelerated. The water-disintegrable composite laminate has a considerably excellent water-disintegradability and also has an excellent mechanical strength in a wet and warm or hot condition.

### INDUSTRIAL APPLICABILITY

As described above, the water-disintegrable resin film, the laminated water-disintegrable resin film and the water-disintegrable composite laminate according to the present invention are each useful as a back sheet of a liner for sanitary goods such as a sanitary napkin liner, a liner for vaginal discharge or a liner for incontinence.

## Claims

1. A water-disintegrable resin film, **characterized in that** it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

2. A water-disintegrable resin film according to claim 1, **characterized in that** it exhibits a tensile strength at 2% elongation of 1 to 40 N/25 mm.

3. A water-disintegrable resin film according to claim 1, **characterized in that** it is a film of 10 to 100 µm in thickness, composed of a polyvinyl alcohol-based resin having a degree of saponification of 95 mol% or more and a degree of polymerization of 300 to 2000.

4. A laminated water-disintegrable resin film produced by integrally laminating a waterproofing layer on one side or both sides of a water-disintegrable resin film of 10 to 100 µm in thickness composed of a polyvinyl alcohol-based resin having a degree of saponification of 95 mol% and a degree of polymerization of 300 to 2000, **characterized in that** it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

5. A laminated water-disintegrable resin film according to claim 4, **characterized in that** the polyvinyl alcohol-based resin is composed of 100 parts by weight of an oxyalkylene-modified polyvinyl alcohol-based resin having an oxyalkylene group content of 1 to 50% by weight, and 25 to 3200 parts by weight of an ethylene/vinyl alcohol copolymer having an ethylene content of 15 mol% or less.

6. A laminated water-disintegrable resin film according to claim 5, **characterized in that** the polyvinyl alcohol-based resin comprises 100 parts by weight of an oxyalkylene-modified polyvinyl alcohol-based resin having an oxyalkylene group content of 1 to 50% by weight, wherein a degree of saponification is 95 mol% or more and a degree of polymerization is 300 to 2000, and 25 to 3200 parts by weight of an ethylene/vinyl alcohol copolymer composed of 30 to 95% by weight of an ethylene/vinyl alcohol copolymer having an ethylene content of 15 mol% or less, wherein a degree of saponification is 95 mol% or more and a degree of polymerization is 300 to 600, and 5 to 70% by weight of an ethylene/vinyl alcohol copolymer having an ethylene content of 15 mol% or less, wherein a degree of saponification is 95 mol% or more and a degree of polymerization is 700 to 2000.

7. A laminated water-disintegrable resin film according to claim 4, **characterized in that** the waterproofing layer is composed of a thermoplastic polyurethane-based resin or a cellulose-based resin.

8. A laminated water-disintegrable resin film according to claim 4, **characterized in that** the waterproofing layer is integrally laminated on the surface of the water-disintegrable resin film through an adhesive layer.

9. A water-disintegrable composite laminate produced by integrally laminating a waterproofing layer on one side of a water-disintegrable resin film of 10 to 100 µm in thickness, composed of a polyvinyl alcohol-based resin having a degree of saponification of 95 mol% or more and a degree of polymerization of 300 to 2000, through an adhesive layer or without through the adhesive layer, and integrally laminating a water-disintegrable backup material on the other side thereof, **characterized in that** it exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.

10. A water-disintegrable resin film according to claim 1, **characterized in that** a degree of biodegradation after 28 days is 30% or more when it is measured nearly according to JIS K 6950.

11. A laminated water-disintegrable resin film according to claim 4, **characterized in that** a degree of biodegradation after 28 days is 30% or more when it is measured nearly according to JIS K 6950.

12. A water-disintegrable composite laminate according to claim 9, **characterized in that** a degree of biodegradation after 28 days is 30% or more when it is measured nearly according to JIS K 6950.

13. A water-disintegrable resin film according to claim 1, which is used as a back sheet of a liner for sanitary goods, wherein the liner is produced by overlapping a liquid-permeable top sheet and the back sheet with each other, integrating opposing outer peripheries of the sheets, and putting a liquid absorbent in a space between opposing faces of the top sheet and the back sheet.

14. A laminated water-disintegrable resin film according to claim 4, which is used as a back sheet of a liner for sanitary goods, wherein the liner is produced by overlapping a liquid-permeable top sheet and the back sheet with each other, integrating opposing outer peripheries of the sheets, and putting a liquid absorbent in a space between opposing faces of the top sheet and the back sheet.

15. A water-disintegrable composite laminate according to claim 9, which is used as a back sheet of a liner for sanitary goods, wherein the liner is produced by overlapping a liquid-permeable top sheet and the back sheet with each other, integrating opposing outer peripheries of the sheets, and putting a liquid absorbent in a space between opposing faces of the top sheet and the back sheet.

16. A method for production of a water-disintegrable composite laminate, comprising: extruding a polyvinyl alcohol-based resin having a degree of saponification of 95 mol% or more and a degree of polymerization of 300 to 2000 and laminating the polyvinyl alcohol-based resin of 10 to 100 µm in thickness on one side of a water-disintegrable backup material, thereby obtaining a laminate produced by integrally laminating a water-disintegrable resin film on the water-disintegrable backup material; and integrally laminating a waterproofing layer on the water-disintegrable resin film side of the laminate by an application method through an adhesive layer formed by a direct application method or a transfer method or without through the adhesion layer, **characterized in that** the water-disintegrable composite laminate exhibits a tensile strength of 2 N/25 mm width or more after the surface thereof is pressed by a cotton gauze impregnated with water of 40°C with a pressure of 1.47 N/cm² for 5 hours under a condition wherein the temperature is 40°C and the relative humidity is 90%, and exhibits a start time for the disintegration thereof by water, which means the time needed until it starts to lose its form, of 600 seconds or less, in a test nearly according to JIS P 4501 for the capability of being united.
